Europäisches Patentamt

European Patent Office

Office européen des brevets

(11) Veröffentlichungsnummer: **0 053 701**
**B1**

(12) EUROPÄISCHE PATENTSCHRIFT

(45) Veröffentlichungstag der Patentschrift:
20.03.85

(51) Int. Cl.⁴: **D 04 H  13/00,** D 04 H  1/46

(21) Anmeldenummer: **81108905.1**

(22) Anmeldetag: **26.10.81**

(54) **Ungewebtes textiles Flächengebilde.**

(30) Priorität: **10.12.80  CH 9086/80**

(43) Veröffentlichungstag der Anmeldung:
**16.06.82 Patentblatt 82/24**

(45) Bekanntmachung des Hinweises auf die Patenterteilung:
**20.03.85 Patentblatt 85/12**

(84) Benannte Vertragsstaaten:
**AT BE CH DE FR GB IT LI LU NL SE**

(56) Entgegenhaltungen:
**EP - A - 0 013 427**
**EP - A - 0 013 428**
**FR - A - 2 299 473**

(73) Patentinhaber: **BREVETEAM S.A., c/o Dr. Paul Stadlin
Gartenstrasse 2 Postfach 758, CH-6300 Zug (CH)**

(72) Erfinder: **Der Erfinder hat auf seine Nennung verzichtet**

(74) Vertreter: **Lesser, Karl-Bolko, Dipl.-Ing., European
Patent Attorney Johanneskirchnerstrasse 149a,
D-8000 München 81 (DE)**

## Beschreibung

Die Erfindung betrifft ein ungewebtes textiles Flächengebilde gemäss Oberbegriff des Anspruches 1.

Es sind bereits textile Flächengebilde der gattungsgemässen Art bekannt (EP-A 0 013 428 und EP-A 0 013 427), bei denen sogenannte Kugelgarne aus sphärisch verwickelten Fasern vorliegen, die jedoch locker in einer nadelfähigen Dichte verwickelt sind. Sie müssen daher durch aktive Vernadelung, d.h. in sich durch aus ihnen entstammende eigene Vernadelungsfasern verfestigt sein, um Eigenfestigkeit zu besitzen. Die aktive Vernadelbarkeit der Kugelgarne wird darüberhinaus auch zur Befestigung derselben an der Trägerschicht verwendet, wobei die Vernadelungsfasern als Haltefasern in die Trägerschicht hineingenadelt sind. Durch die aktive Vernadelung werden die Kugelgarne jedoch mehr oder weniger zusammengedrückt, was insbesondere bei dichter Vernadelung noch verstärkt wird. Dieses führt zu einem abgeplatteten, eher filzartig verformten Gebilde, so dass die durch die Kugelgarne gegebenenfalls gewünschte Struktur, insbesondere Hoch-Tiefstruktur, beeinträchtigt wird und nicht in dem gewünschten Aussehen vorliegen kann. Lediglich zusätzlich können bei Vorliegen einer aktiv nadelfähigen Trägerschicht die auch passiv nadelfähigen, jedoch durch die aktive Vernadelung verfestigten Kugelgarne von der Trägerschicht her vernadelt sein. Dieses beseitigt jedoch nicht die vorstehend genannten Nachteile der bekannten Flächengebilde, so dass sie daher nicht allen Anforderungen des Verarbeitens und des Benutzers gerecht werden.

Aus der FR-A 2 299 473 bzw. DE-A 2 604 098 sind Bodenbeläge bekannt, bei denen mindestens eine Lage von parallel nebeneinander angeordneten Textilfäden von den Fäden her durch aus diesen entstammende Fasern mit einer Stützschicht vernadelt sind. Da die Vernadelung durch Fasern der Fäden, d.h. von der Oberseite des Bodenbelages her erfolgt, ist wegen der Gefahr der Zerstörung der gedrehten Fäden und damit des Aussehens derselben jedoch nur ein geringer Teil der Fasern jedes Fadens an der Trägerschicht mit einer ungenügenden Dichte vernadelt. Als Vernadelungsdichte wird dort eine Stichdichte von 0,3 bis 6 Einstichen/cm² angegeben. Hierdurch liegt eine für viele Zwecke ungenügende Festigkeit der Fadenschicht an der Stützschicht vor, die bei Beschädigung der aus den Fäden stammenden Fasern bei Benutzung des Bodenbelages zu einer Abtrennung der Fadenlage und damit zu einer Zerstörung der Oberseite des Bodenbelages führen kann. Will man auf der Grundlage dieses bekannten Verfahrens eine dichtere Vernadelung durchführen, so führt dies allenfalls zu einem flachen, filzartig verformten Gebilde, für das sich ein Herstellen von einzelnen Fäden nicht lohnt, auch wenn ein strukturiertes Aussehen damit erzielt werden kann. Diese bekannten Beläge entsprechen daher nicht den an sie gestellten Anforderungen und sind auf Fäden, die eine geringe Vernadelungsdichte zulassen und damit auf Beläge geringer Dicke beschränkt. Abschnitte einer weiteren Fadenlage über der ersten Fadenlage führen bei diesen bekannten Belägen darüberhinaus lediglich zu einer zusätzlichen Musterung.

Der Erfindung liegt deshalb die Aufgabe zugrunde, ein gattungsgemässes ungewebtes textiles Flächengebilde ohne die Nachteile der bekannten Flächengebilde zu schaffen, bei dem die faserhaltigen Kugeln an dessen Oberseite mit unverändert runder Gestalt vorliegen und mit ausreichender Festigkeit an der Trägerschicht befestigt sein sollen, derart, dass ein deutlich strukturiertes Aussehen, z.B. eine erhabene Struktur, ungestört durch die Befestigungsart erreicht sein soll.

Diese Aufgabe wird durch den Gegenstand des Anspruches 1 gelöst. Das gattungsgemässe Flächengebilde ist erfindungsgemäss dadurch gekennzeichnet, dass die Kugeln ohne gegenseitigen Zusammenhalt vorliegen und sich die aus der Trägerschicht stammenden Haltefasern von aussen unsichtbar lediglich in die Kugeln hinein bis höchstens zu der der Trägerschicht abgewandten Peripherie der Kugeln erstrecken und innerhalb der Kugeln mit einem Endteil in Richtung der Trägerschicht umgebogen vorliegen, so dass die Kugeln durch die lediglich eingedrungenen Haltefasern von der Trägerschicht her lediglich passiv vernadelt vorliegen.

Die Kugeln sind vorzugsweise durch einen üblichen Vernadelungsprozess an der Trägerschicht befestigt, bei dem von der Trägerschicht her z.B. mittels Nadeln mit Widerhaken Fasern der Trägerschicht erfasst und als Haltefasern in die Kugeln eingeführt und diese somit passiv vernadelt werden können. Die passive Befestigung der Kugeln, bzw. die Ausbildung der Haltefasern kann aber auch durch andere bekannte Verfahren, z.B. die Maliverfahren, erfolgt sein.

Auch nachfolgend ist unter «aktiv vernadelt» zu verstehen, dass Fasern einer Faserschicht durch einen Vernadelungsprozess relativ zu anderen Fasern in derselben bewegt, z.B. ausgezogen, und miteinander verschlungen werden, d.h. aktiv etwas bewirken können. Sie können in eine an die Faserschicht angrenzende weitere Schicht eingebracht sein, so dass die weitere Schicht mit der Faserschicht verbunden ist. Unter «passiv vernadelt» bzw. «passiv befestigt» ist dagegen zu verstehen, dass sich in einer ebenfalls faserhaltigen Materialschicht Fasern aus einer anderen faserhaltigen Schicht als Haltefasern befinden, jedoch die Fasern dieser Materialschicht dabei im wesentlichen nicht für aktive Zwecke herangezogen, sondern passiv verblieben sind. Hierzu wird z.B. auch auf die schweizerische Patentschrift 493 206 verwiesen.

Die auf der Oberfläche des erfindungsgemässen textilen Flächengebildes vorliegenden Kugeln liegen daher auf einer aktiv vernadelten Trägerschicht vor, von deren Fasern ein Teil als die Haltefasern in die Kugeln lediglich eingedrungen sind, so dass die Kugeln durch die aktive Beteiligung der Fasern aus der Trägerschicht daher passiv befestigt sind. Die gegeneinander verfestigten Fasern oder Fäden werden z.B. im wesentlichen ähnlich wie durch die Fasereinbindung in einem gesponnenen Garn festgehalten und sind daher aktiv unvernadelbar oder im wesentlichen aktiv unvernadelbar. Die Kugeln sind vorzugsweise in sich bereits verfestigt und besitzen eine Eigenfestigkeit und auch bei Gebrauch des Flächengebildes ausreichende und gewünschte Haltbarkeit, so dass sich eine Verfestigung durch aktive Vernadelung sich nicht nur erübrigt, sondern darüberhinaus im wesentlichen auch behindert, wenn nicht unmöglich ist. Die Befestigung durch Vernadelung kann daher in vorteilhafter Weise von der Trägerschicht her lediglich passiv vorgenommen sein. Die Kugeln liegen unbeeinflusst durch die Befestigung mit im wesentlichen unveränderter runder Gestalt, z.B. unverändert kreisförmigen Querschnitt, vor, so dass eine deutlich gegenüber dem Grund, d.h., der Trägerschicht, erhabene Struktur erreicht werden kann. Es kann somit ein Aussehen mit deutlich erhabener Struktur erhalten werden, das sich deutlich von demjenigen der eingangs geschilderten bekannten textilen Flächengebidle unterscheidet, die durch die aktive Vernadelung zur Verfestigung der Kugelgarne ein filzartiges Aussehen aufweisen.

Die Haltefasern erstrecken sich von aussen unsichtbar lediglich bis höchstens zu der der Trägerschicht abgewandten Peripherie derselben in die Kugeln hinein. Gemäss einer Ausführungsform der Erfindung erstrecken sich die Haltefasern nur auf einem Teil der Dicke der Kugeln, z.B. der halben Dicke derselben, in diese hinein. Hierdurch ist die Oberseite des Flächengebildes frei oder im wesentlichen frei von herausstehenden Haltefasern, so dass diese keiner Abnutzung ausgesetzt sind und bei Benutzung des Flächengebildes keine Beschädigung erfahren können. Die Befestigung der Kugeln an der Trägerschicht kann daher unverändert aufrechterhalten werden. Die aus der passiven Vernadelung hervorgerufenen Nadeleinstiche liegen an der Trägerschicht und damit an der Unterseite des Flächengebildes vor und sind daher an der Oberseite des Flächengebildes dem Blick des Betrachters entzogen und unsichtbar. Die Kugeln sind daher durch eine sogenannte «Blindstichtechnik», d.h. unsichtbare Technik, aus der aktiv nadelfähigen Trägerschicht vernadelt, so dass sie durch Gebrauch nicht beeinflusst werden können. Erfindungsgemäss sind die Kugeln daher ohne Gefahr ihrer Zerstörung und der Beeinflussung ihres Aussehens befestigt, da ihre Fasern selbst nicht zum Befestigen an der Trägerschicht herangezogen sind.

Die Haltefasern sind mit einem Endteil innerhalb der Kugeln in Richtung der Trägerschicht umgebogen. Sie können hakenförmig, z.B. widerhakenförmig, n-förmig, umgekehrt V-förmig oder schlingen- oder schlaufenförmig umgebogen sein. Durch diese Umbiegung können die Haltefasern mit ihrem einen Ende mit den Fasern der Kugeln verhakt und daher fest in denselben verankert sein. Mit ihrem anderen Ende befinden sich die Haltefasern jedoch in der Trägerschicht, wobei bei z.B. schlaufenförmiger Umbiegung auch beide Enden in der Trägerschicht verankert sein können.

Durch die Kugeln liegt z.B. auch ein abgeschlossenes Gebilde vor, das infolge seiner Dicke von mindestens 6 mm, die bis zu 50 mm betragen kann, an mehreren Stellen parallel über den Querschnitt gesehen von den Haltefasern und daher mit hoher Gleichmässigkeit festgehalten werden kann, so dass die Kugeln mit ausreichender gewünschter Festigkeit an der Trägerschicht befestigt werden können. Es ist daher ohne Störung oder wesentliche Änderung der Gestalt der Kugeln auch eine hohe Vernadelungsdichte und damit ein passives Kompaktieren möglich, wodurch z.B. die Festigkeit und Gleichmässigkeit der Befestigung erhöht wird. Je nach Art der Kugeln, des gewünschten Flächengebildes oder der gewünschten oder notwendigen Festigkeit können die Kugeln von der Trägerschicht her mit einer Nadeldichte von 20 bis 200 Einstichen pro $cm^2$ vernadelt sein. Infolge der Kugelgarndicke von mehr als 6 mm können textile Flächengebilde mit einem Gewicht von 0,5 bis 8 kg/$m^2$ vorliegen.

Beim erfindungsgemässen Flächengebilde ist bereits mit einer einzigen Lage aus den Kugeln ein dickes Produkt möglich, bei dem die Kugeln deutlich erhaben vorliegen können und als dreidimensionale Gebilde vom Grund, d.h. von der Trägerschicht, abstechend erkennbar sind. Es entfällt das Anfertigen eines Vormusters. Die Kugeln können vielmehr einzeln und in einer gewünschten Musterung auf die Trägerschicht aufgebracht sein, z.B. dicht nebeneinander oder mit Abstand voneinander, wodurch nicht nur das Aufbringen derselben erleichtert werden kann, sondern auch eine Vielfalt von Musterungen oder Strukturen und ein Wechsel derselben ermöglicht werden kann. Durch streifenförmige Anordnung der Kugeln kann ein web- oder tuftingähnlicher Charakter vorgetäuscht werden.

Zwischen den Kugeln kann das Flächengebilde beim Ab- oder Umbiegen in Richtung der Trägerschicht aufklaffen, ohne dass dabei die Befestigung der Kugeln an der Trägerschicht beeinträchtigt oder beansprucht wird. Hierdurch erhält das Flächengebilde z.B. eine Geschmeidigkeit, wie sie z.B. bei einem vernadelten Filz oder bei den bekannten Flächengebilden der eingangs geschilderten EP-Veröffentlichungen nicht erreicht werden kann, so dass ähnliche Eigenschaften wie z.B. bei getufteten Teppichen vorliegen.

Die Fasern oder Fäden der Kugeln können zu ihrer Verfestigung dicht verschlungen, verklebt, durch aufgeschäumtes Material gebunden oder verfilzt vorliegen. Sie können als filzartige Klümpchen oder wie z.B. beim Spinnprozess erzeugte noppenartige Gebilde vorliegen. Hierdurch sind die Fasern daran gehindert, aus dem Faserverband der Kugeln z.B. durch Vernadeln aktiv ausgezogen zu werden, zumindest kann ihre gegenseitige Bewegungsfähigkeit dadurch erschwert oder beschränkt werden. Die Fasern oder Fäden sind daher z.B. derart blockiert, dass sie zumindest nicht mehr genügend gegeneinander gleiten und daher bei der Vernadelung nicht genügenden Nachschub für die Nadel geben und nicht herausgezogen werden können. Bei einem Versuch, die verfestigten Fasern oder Fäden aktiv zu vernadeln, würden dieselben ausgestanzt werden oder reissen oder die Nadeln würden brechen. Die Kugeln sind daher lediglich von den eingedrungenen Haltefasern durchsetzt, ohne dass ihre Fasern oder Fäden in irgendeiner Weise aktiv bei der Befestigung an der Trägerschicht teilgenommen haben. In einer Ausführungsform können die Fasern oder Fäden der Kugeln in einer Dichte von 0,1 bis 0,25 g/cm$^3$ vorliegen, wodurch ein kompaktes Kugelgebilde mit günstiger Verfestigung der Fasern oder Fäden erreicht werden kann. Infolge der erfindungsgemäss eingedrungenen Haltefasern, die unter Spannung oder locker in den Kugeln vorliegen können, wird z.B. auch ein Zusammendrücken der Kugeln und damit ein abgeplattetes Produkt vermieden, wie es z.B. beim Vernadeln von oben her, insbesondere bei hoher Vernadelungsdichte bei den bekannten Flächengebilden auftritt.

Die Kugeln können Kugelgarne aus sphärisch verwickelten Fasern oder Fäden sein und eine kugelförmige oder kugelartige, z.B. wurmförmige, ovale oder zylindrische Form aufweisen. Sie können gleiche oder voneinander verschiedene Beschaffenheit, z.B. in Grösse, Farbe, Gestalt, Faserart oder dergleichen besitzen und die Fasern oder Fäden der Kugeln oder Kugelgarne können natürliche oder synthetische Fasern oder ein Gemisch derselben sein bzw. enthalten, wobei die Fäden auch Filamente sein können. Die Fasern oder Fäden der Kugelgarne können eine Länge von 40 bis 5000 mm besitzen. Bei kugelförmiger Gestalt können die Kugeln eine Dicke von 6 bis 25 mm und bei z.B. wurmartiger Form eine Dicke von 4 bis 15 mm aufweisen.

Weitere Eigenschaften der Kugelgarne, wie sie für das Flächengebilde nach der Erfindung von Vorteil sein können, sind in den bereits erwähnten EP-Veröffentlichungen beschrieben, weshalb an dieser Stelle darauf verwiesen wird. Die erfindungsgemässe Befestigung kann somit in günstiger Weise mit den Eigenschaften der Kugelgarne kombiniert werden, so dass das erfindungsgemässe Flächengebilde die unveränderten Eigenschaften der erfindungsgemäss eingesetzten Kugelgarne an seiner Oberseite aufweisen kann.

Das textile Flächengebilde kann in Form einer Bahn oder in Abschnitten, z.B. als Tafel, oder als Stückware, vorliegen und es kann ein Boden- oder Wandbelag, ein Dekorations-, Möbel- oder Bekleidungsstoff oder eine Decke sein oder für solche verwendet werden.

Die Herstellung des erfindungsgemässen Flächengebildes erfordert eine gezielte Hineinnadelung der Haltefasern aus der Trägerschicht heraus in die Kugeln hinein. Dies erfolgt vorzugsweise mit Nadeln, die sowohl ein Erfassen, als auch ein ungestörtes Freigeben der z.B. schlingenförmig von Widerhaken oder Ausnehmungen an den Nadeln erfassten Haltefasern beim Zurückziehen der Nadeln aus den Kugeln ermöglichen, so dass die Haltefasern innerhalb der Kugeln mit ihrem vorzugsweise umgebogenen Ende vorliegen können. Das Vernadeln kann mit sogenannten Close-barb-Nadeln erfolgen (z.B. Informationsblatt F01A, Singer Filznadeln). Hierbei sind z.B. die Abstände von barb zu barb, d.h. der Widerhaken, sowie z.B. die Durchstichtiefe und/oder Stichdichte dafür massgebend, ob Einzelfasern oder Büschel von einem Widerhaken erfasst werden können und wie tief und in welchem Abstand dieselben in den Kugeln positioniert und z.B. nach Art einer Schlaufe umgebogen angebracht sein können. Durch entsprechende Anordnung der Nadeln im Nadelbrett und Lage der Kugeln, z.B. nebeneinander mit Freiräumen dazwischen, können die Kugeln in gewünschter Weise vernadelt werden, so dass in gezielter Weise die Haltefasern lediglich in die Kugeln eingedrungen vorliegen.

Die Trägerschicht kann natürliche oder synthetische Fasern enthalten oder aus denselben bestehen und z.B. ein Faserverbundstoff sein. Für die Trägerschicht kann auch von einem Faservlies oder einer Faserlage, z.B. aus parallel oder gekreuzt liegenden Fasern oder Fäden ausgegangen werden. An der den Kugeln abgewandten Seite der Trägerschicht kann eine Unterlagsschicht, z.B. eine für Bodenbeläge übliche Abdeckschicht an der Rückseite, z.B. eine Federrückenschicht oder Bindemittelschicht, durch Vernadelung oder Verklebung befestigt sein, um den Belag den gewünschten Anforderungen gemäss auszubilden oder auch zur Sicherung der Haltefasern an der Unterseite.

Bei dem erfindungsgemässen textilen Flächengebilde können daher in überraschender Weise Eigenschaften von Kugeln, z.B. Kugelgarnen aus sphärisch verwickelten Fasern, mit dem passiven Befestigen der Kugeln an der Trägerschicht gemäss der Erfindung vereint werden und ein Produkt mit unveränderten Eigenschaften und insbesondere Gestalt der Kugeln an der Oberseite erhalten werden, was bereits mit einer einzigen Lage der nebeneinanderliegenden Kugeln auf der Trägerschicht erreicht werden kann. Die Kugeln können auch mit Bindemitteln, die von der Trägerschicht her eingebracht werden und von dort her in dieselben vordringen können, d.h. durch sogenanntes Pflatschen oder durch Tränken, behandelt sein.

Die Erfindung ist nachfolgend anhand von in der Zeichnung dargestellten Ausführungsbeispielen näher erläutert.

Es zeigt:

Fig. 1 einen Teil eines ungewebten textilen Flächengebildes in schaubildlicher, schematischer Darstellung mit einem Schnitt, und

Fig. 2 einen Teil eines ungewebten textilen Flächengebildes im Schnitt in schematischer Darstellung.

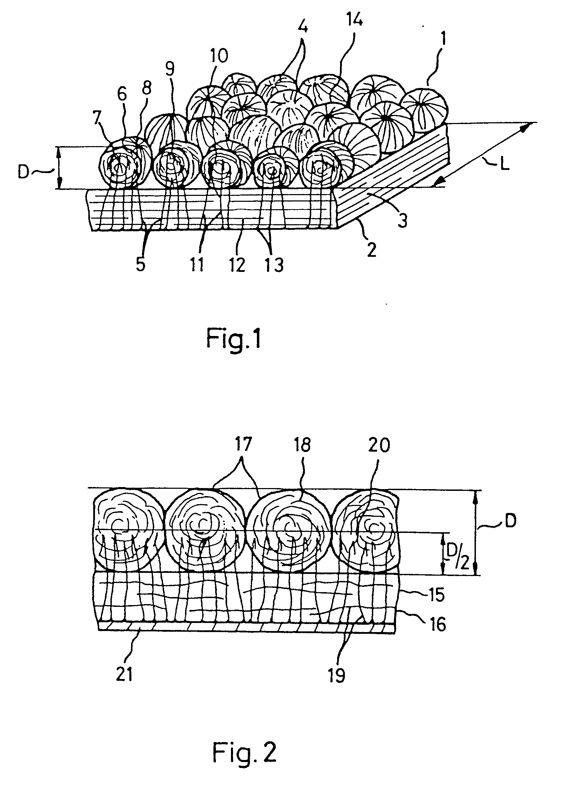

Das ungewebte textile Flächengebilde nach Fig. 1 ist z.B. ein Bodenbelag 1, z.B. ein Teppich, und besitzt eine ungewebte Trägerschicht 2 aus einem Fasermaterial 3, auf der dicke Kugeln 4 vorliegen, die eine Dicke D von mehr als 6 mm aufweisen. Die Trägerschicht 2 ist aktiv mit den Kugeln 4 vernadelt, d.h., die Kugeln 4 sind durch aus der Trägerschicht 2 stammende Haltefasern 5 auf derselben befestigt und damit passiv vernadelt. Die Haltefasern 5 erstrecken sich lediglich in die Kugeln 4 hinein und bis oder im wesentlichen bis an die Peripherie 6 der Kugeln 4 bzw. Abschnitten der Peripherie 6, die der Trägerschicht 2 abgewandt ist. An ihrem in die Kugeln 4 lediglich eingedrungenen Teil 7 sind die Haltefasern 5 umgebogen und können somit in den Fasern 8 der Kugeln 4 verhakt und damit befestigt sein. Der umgebogene Faserteil 7 kann ein Faserendteil 9 oder eine Faserschlaufe 10 sein, die mit ihren beiden Enden 11 in der Trägerschicht 2 verblieben vorliegt, was von der Stapellänge des Fasermaterials 3 der Trägerschicht 2 oder dem beim Vernadeln gerade an der Nadel vorliegenden Faserteil abhängt. Zur Verdeutlichung sind die umgebogenen Faserteile 7 bzw. 10 breiter dargestellt, als sie tatsächlich vorliegen können, d.h. sie sind praktisch enger.

Die Kugeln 4 liegen als Kugelgarne vor, in denen die Fasern 8 sphärisch verwickelt sind, wie es z.B. in den bereits vorstehend erwähnten EP-Veröffentlichungen geschildert ist. Jedoch sind die Fasern 8 in einer Dichte von z.B. 0,1 bis 0,25 g/cm³ sphärisch verschlungen, so dass sie verfestigt und daher aktiv unvernadelbar sind. An der Unterseite 12 der Trägerschicht 2 und damit des Bodenbelages 1 liegen Einstiche 13 vor, die beim Vernadeln der Trägerschicht 2 mit den Kugeln 4 entstanden und die von der Oberseite des Belages 1 her unsichtbar sind. Durch die passive Vernadelung von der Trägerschicht 2 her sind die Kugeln 4 unabgeplattet und haben ihre volle ursprüngliche Dicke D und durch die lediglich in die Kugeln 4 eingedrungenen Haltefasern 5 ist die Oberseite des Belages 1 daher frei von Haltefasern 5.

Die Kugeln 4 sind durch die Haltefasern 5 mit genügender Festigkeit an der Trägerschicht 2 angeheftet, weil die Vernadelung an einer Vielzahl von Vernadelungsstellen entsprechend den Einstichen 13 erfolgt ist. Zur Herstellung werden die Kugeln 4 einzeln auf die Trägerschicht 2 aufgebracht und liegen nach dem passiven Vernadeln ohne gegenseitigen Zusammenhalt vor, so dass der Bodenbelag 1 z.B. quer zur Längsrichtung L unter Aufklaffen von Zwischenräumen zwischen den Kugeln 4 biegbar und daher z.B. in seiner Querrichtung flexibel ist.

Bei dem ungewebten textilen Flächengebilde 15 nach Fig. 2, das wiederum ein Bodenbelag sein kann, liegt eine Trägerschicht 16 vor, auf der Kugelgarne 17 aus sphärisch verwickelten Fasern 18 durch aus der Trägerschicht 16 stammende Haltefasern 19 befestigt sind. Die Fasern 18 liegen wiederum in einer Dichte von 0,1 bis 0,25 g/cm³ vor, d.h. verfestigt und aktiv unvernadelbar, so dass die Kugelgarne 17 wiederum passiv durch die Haltefasern 19 vernadelt sind. Mit einem umgebogenen Teil 20 erstrecken sich die Haltefasern 19 wiederum lediglich in die Kugelgarne 17 hinein, und zwar auf der halben Dicke D/2 der Kugelgarne 17, und sind in den Fasern 18 derselben verankert. An der Unterseite des Flächengebildes 15 ist eine Abdeckschicht 21 angebracht, durch welche die von der Trägerschicht 16 ausgehenden Haltefasern geschützt sind.

## Patentansprüche

1. Ungewebtes textiles Flächengebilde (1, 15) mit einer Oberschicht aus faserhaltigen Kugeln (4, 17), die eine Dicke von mindestens 6 mm aufweisen, und mit einer faserhaltigen Trägerschicht (2, 16), bei dem die Kugeln (4, 17) mittels als Haltefasern (5, 19) vorliegenden Fasern mit der Trägerschicht (2, 16) vernadelt sind, dadurch gekennzeichnet, dass die Kugeln (4, 17) ohne gegenseitigen Zusammenhalt vorliegen und sich die aus der Trägerschicht (2, 16) stammenden Haltefasern (5, 19) von aussen unsichtbar lediglich in die Kugeln (4, 17) hinein bis höchstens zu der der Trägerschicht (2, 16) abgewandten Peripherie der Kugeln (4, 17) erstrecken und innerhalb der Kugeln (4, 17) mit einem Endteil (5') in Richtung der Trägerschicht (2, 16) umgebogen vorliegen, so dass die Kugeln (4, 17) durch die lediglich eingedrungenen Haltefasern (5, 19) von der Trägerschicht (2, 16) her lediglich passiv vernadelt vorliegen.

2. Flächengebilde nach Anspruch 1, dadurch gekennzeichnet, dass sich die Haltefasern (5, 19) nur auf einem Teil der Dicke der Kugeln (4, 17), z.B. auf der halben Dicke, in dieselben hineinerstrecken.

3. Flächengebilde nach Anspruch 1 oder 2, dadurch gekennzeichnet, dass die in die Kugeln (4, 17) eingedrungenen Haltefasern (5, 19) hakenförmig, z.B. widerhakenförmig, n-förmig, umgekehrt V-förmig oder schlingen- oder schlaufenförmig umgebogen vorliegen.

4. Flächengebilde nach einem der vorhergehenden Ansprüche, dadurch gekennzeichnet, dass die Kugeln (4, 17) eine Dicke von bis zu 50 mm aufweisen.

5. Flächengebilde nach einem der vorhergehenden Ansprüche, dadurch gekennzeichnet, dass die Fasern oder Fäden der Kugeln (4, 17) durch dichte Verschlingung oder Verwicklung, Zusammenpressung, Verklebung, Bindung durch aufgeschäumtes Material oder Verfilzung gegeneinander verfestigt aktiv unvernadelbar vorliegen.

6. Flächengebilde nach einem der vorhergehenden Ansprüche, dadurch gekennzeichnet, dass die Fasern (8, 18) oder Fäden der Kugeln (4, 17) in einer Dichte von 0,1 bis 0,25 g/cm³ vorliegen.

7. Flächengebilde nach einem der vorhergehenden Ansprüche, dadurch gekennzeichnet, dass die Kugeln (4, 17) Kugelgarne aus sphärisch verwickelten Fasern oder Fäden sind.

8. Flächengebilde nach Anspruch 7, dadurch gekennzeichnet, dass die Kugelgarne (4, 17) eine kugelförmige oder kugelartige, z.B. wurmförmige, ovale oder zylindrische Form aufweisen.

9. Flächengebilde nach Anspruch 7 oder 8, dadurch gekennzeichnet, dass die Kugelgarne gleiche oder voneinander verschiedene Beschaffenheit, z.B. in Grösse, Farbe, Gestalt, Faserart oder dergleichen, besitzen.

10. Flächengebilde nach einem der Ansprüche 7–9, dadurch gekennzeichnet, dass die sphärisch verwickelten Fasern oder Fäden der Kugelgarne (4, 17) eine Länge von 40 bis 5000 mm aufweisen.

11. Flächengebilde nach einem der vorhergehenden Ansprüche, dadurch gekennzeichnet, dass die Trägerschicht (2, 16) mit den Kugeln (4, 17) mit einer Nadeldichte von 20 bis 200 Stichen/cm² vernadelt ist.

12. Flächengebilde nach einem der vorhergehenden Ansprüche, dadurch gekennzeichnet, dass ein Gewicht des Flächengebildes von 0,5 bis 8 kg/cm² vorliegt.

13. Flächengebilde nach einem der vorhergehenden Ansprüche, dadurch gekennzeichnet, dass die Trägerschicht (2, 16) in sich und mit den Kugeln (4, 17) durch die aus ihr stammenden eigenen Haltefasern (5, 19) aktiv vernadelt ist.

14. Flächengebilde nach einem der vorhergehenden Ansprüche, dadurch gekennzeichnet, dass die Haltefasern an mehreren Stellen parallel im Querschnitt der Kugeln (4, 17) gesehen in dieselben eingedrungen vorliegen.

15. Flächengebilde nach einem der vorhergehenden Ansprüche, dadurch gekennzeichnet, dass eine einzige Lage von nebeneinander liegenden Kugeln (4, 17) vorliegt.

16. Flächengebilde nach einem der vorhergehenden Ansprüche, dadurch gekennzeichnet, dass es ein Boden- oder Wandbelag, ein Dekorations-, Möbel- oder Bekleidungsstoff oder eine Decke ist.

**Claims**

1. Non-woven textile sheet material (1, 15) comprising an upper layer of fibre-containing balls (4, 17) having a thickness of at least 6 mm, and a fibre-containing supporting layer (2, 16) to which the balls (4, 17) are needled by means of fibres, present as retaining fibres (5, 19) from the supporting layer (2, 16), characterised in that the balls (4, 17) are present without mutual interconnection and the retaining fibres (5, 19) originating from the supporting layer (2, 16) extend only into the balls (4, 17), in a manner invisible from outside, at the most as far as the periphery of the balls (4, 17) which faces away from the supporting layer (2, 16), and are present within the balls (4, 17), with an end portion (5′) which is folded back in a direction towards the supporting layer (2, 16), so that the balls (4, 17) are only present passively needled by the retaining fibres (5, 19) simply forced into from the supporting layer (2, 16).

2. Sheet material according to claim 1, characterised in that the retaining fibres (5, 19) extend into the balls (4, 17) only by a portion of their thickness, for example to half the thickness.

3. Sheet material according to claim 1 or 2, characterised in that the retaining fibres (5, 19) forced into the balls (4, 17) are present in a hook-form, for example barbed, n-shaped, inverted V shaped or of open or closed loop form.

4. Sheet material according to one of the foregoing claims, characterised in that the balls (4, 17) have a thickness of up to 50 mm.

5. Sheet material according to one of the foregoing claims, characterised in that the fibres of threads of the balls (4, 17) are held together in a form which is not capable of being actively needled by virtue of tight looping or winding, compression, adhesion, bonding by foam material or knitting.

6. Sheet material according to one of the foregoing claims, characterised in that the fibres (8, 18) or threads of the balls (4, 17) are present in a density of from 0.1 to 0.25 g/cm³.

7. Sheet material according to one of the foregoing claims, characterised in that the balls (4, 17) are ball yarns of spherically wound fibres or threads.

8. Sheet material according to one of the foregoing claim, characterised in that the ball yarns (4, 17) have a ball-shape or ball-like shape, e.g. vermicular, oval or cylindrical form.

9. Sheet material according to one of the two preceding claims, characterised in that the ball yarns have the same or mutually different characteristics, e.g. in size, colour, shape, kind of fibre or the like.

10. Sheet material according to one of the three preceding claims, characterised in that the spherically wound fibres or threads of the ball yarns (4, 17) have a length of 40 to 5000 mm.

11. Sheet material according to one of the foregoing claims, characterised in that the supporting layer (2, 16) is needled to the balls (4, 17) with a needle density of from 20 to 200 needles/cm².

12. Sheet material according to one of the foregoing claims, characterised in that a weight of sheet material of from 0.5 to 8 kg/cm² is present.

13. Sheet material according to one of the foregoing claims, characterised in that the supporting layer (2, 16) is actively needled in itself and to the balls (4, 17) by the retaining fibres (5, 19) originating from itself.

14. Sheet material according to one of the foregoing claims, characterised in that the retaining fibres are present engaged in the balls (4, 17) at several points parallel in the cross section of the balls.

15. Sheet material according to one of the foregoing claims, characterised in that a single layer of mutually adjacent balls (4, 17) is present.

16. Sheet material according to one of the foregoing claims, characterised in that it is a floor covering or wall covering, a decorative material, or furnishing fabric or clothing fabric or a blanket.

## Revendications

1. Produit superficiel textile non tissé (1, 15) comprenant une couche supérieure de nodules (4, 17) contenant des fibres et ayant une épaisseur d'au moins 6 mm, ainsi qu'une couche de soutien (2, 16) contenant des fibres et dans laquelle les nodules (4, 17) sont piqués avec la couche de soutien (2, 16) au moyen de fibres présentes en tant que fibres de retenue (5, 19), caractérisé en ce que les nodules (4, 17) sont disposés sans cohésion réciproque et en ce que les fibres de retenue (5, 19) provenant de la couche de soutien (2, 16) s'étendent, à l'état invisible de l'extérieur, exclusivement à l'intérieur des nodules (4, 17) jusqu'à, au maximum, la périphérie des nodules (4, 17) éloignée de la couche de soutien (2, 16) et sont présentes à l'état replié à l'intérieur des nodules (4, 17) par une partie d'extrémité (5') dans la direction de la couche de soutien (2, 16), si bien que les nodules se présentent exclusivement à l'état piqué passivement à partir de la couche de soutien (2, 16) par les fibres de retenue (5, 19) exclusivement introduites.

2. Produit superficiel selon la revendication 1, caractérisé en ce que les fibres de retenue (5, 19) ne s'étendent à l'intérieur des nodules (4, 17) que sur une partie de leur épaisseur, par exemple, sur la moitié de leur épaisseur.

3. Produit superficiel selon la revendication 1 ou 2, caractérisé en ce que les fibres de retenue (5, 19) introduites dans les nodules (4, 17) se présentent sous la forme d'un crochet, par exemple, un ardillon, un n, un V inversé, une boucle ou un nœud coulant.

4. Produit superficiel selon l'une des revendications précédentes, caractérisé en ce que les nodules (4, 17) ont une épaisseur de 6 à 50 mm.

5. Produit superficiel selon l'une des revendications précédentes, caractérisé en ce que les fibres ou fils des nodules (4, 17) se présentent à l'état serré réciproquement et activement non piquable par un enroulement ou entremêlement dense, un pressage en commun, un collage, une liaison par une matière expansée ou un feutrage.

6. Produit superficiel selon l'une des revendications précédentes, caractérisé en ce que les fibres (8, 18) ou fils des nodules (4, 17) ont une densité de 0,1 à 0,25 g/cm³.

7. Produit superficiel selon l'une des revendications précédentes, caractérisé en ce que les nodules (4, 17) sont des filés nodulaires à base de fibres ou de fils enroulés en sphères.

8. Produit superficiel selon la revendication 7, caractérisé en ce que les filés nodulaires (4, 17) sont sous la forme d'un nodule ou semblable à un nodule, par example, une forme vermiculaire, ovale ou cylindrique.

9. Produit superficiel selon la revendication 7 ou 8, caractérisé en ce que les filés nodulaires ont une nature égale ou différente les unes des autres, par example, en grandeur, couleur, forme, type de fibres ou analogues.

10. Produit superficiel selon l'une des revendications 7 à 9, caractérisé en ce que les fibres ou fils enroulés en sphères des filés nodulaires (4, 17) ont une longueur de 40 à 5000 mm.

11. Produit superficiel selon l'une des revendications précédentes, caractérisé en ce que la couche de soutien (2, 16) est piquée avec les nodules (4, 17) selon une densité de 20 à 200 piqûres/cm².

12. Produit superficiel selon l'une des revendications précédentes, caractérisé en ce que le poids du produit superficiel est de 0,5 à 8 kg/cm².

13. Produit superficiel selon l'une des revendications précédentes, caractérisé en ce que la couche de soutien (2, 16) est piquée activement en soi et avec les nodules (4, 17) par les fibres de retenue propres (5, 19) provenant de ladite couche de soutien.

14. Produit superficiel selon l'une des revendications précédentes, caractérisé en ce que les fibres de retenue sont introduites dans les nodules (4, 17) parallèlement à plusieurs endroits, en considérant la section droite de ces nodules.

15. Produit superficiel selon l'une des revendications précédentes, caractérisé en ce qu'une seule couche de nodules (4, 17) disposés côte à côte est présente.

16. Produit superficiel selon l'une des revendications précédentes, caractérisé en ce qu'il consiste en un revêtement de sol ou de mur, une étoffe de décoration, d'ameublement ou d'habillement ou de couverture.

Fig.1

Fig. 2